# EUROPÄISCHE PATENTANMELDUNG

(11) **EP 4 091 560 A1**
(43) Veröffentlichungstag der Anmeldung: **23.11.2022**
(21) Anmeldenummer: 22174453.5
(22) Anmeldetag: 19.05.2022
(51) Int. Cl.: A61B 17/66

(54) **DISTRAKTIONSSYSTEM**

(30) Priorität: 21.05.2021 DE 102021113317
(71) Anmelder: Technische Universität Dresden, 01069 Dresden (DE)
(72) Erfinder: BOTZENHART, Ute Ulrike, 01309 Dresden (DE)
(74) Vertreter: Riechelmann & Carlsohn Patentanwälte PartG mbB

(57) **Zusammenfassung**

Die Erfindung betrifft ein Distraktionssystem, insbesondere zur transversalen Erweiterung eines Kiefers, aufweisend ein erstes Fixierungselement (11) zur Befestigung am Kiefer sowie ein zweites Fixierungselement (12) zur Befestigung am Kiefer, wobei das Distraktionssystem (1) ferner ein erstes Brückenelement (21) zur lösbaren Befestigung an dem ersten Fixierungselement (11), ein zweites Brückenelement (22) zur lösbaren Befestigung an dem zweiten Fixierungselement (12) und einen Distraktionszylinder (31) zum Verändern des Abstandes zwischen dem ersten Brückenelement (21) und dem zweiten Brückenelement (22) aufweist. Dabei ist vorgesehen, dass das Distraktionssystem (1) ferner einen Konsolidierungssteg (51) zur lösbaren Befestigung an dem ersten Fixierungselement (11) und dem zweiten Fixierungselement (12) unter Ersatz des ersten Brückenelementes (21), des zweiten Brückenelementes (22) und des Distraktionszylinders (31) aufweist.

## Beschreibung

Die Erfindung betrifft ein Distraktionssystem, insbesondere ein Distraktionssystem zur transversalen Erweiterung eines Kiefers. Sie betrifft ferner ein Distraktionsset zur Herstellung einer Distraktionseinrichtung, insbesondere zur Herstellung einer Distraktionseinrichtung zur transversalen Erweiterung eines Kiefers, und zur Herstellung einer Konsolidierungseinrichtung.

Die Unterkiefersymphyse besteht aus zwei paarigen Anteilen, die bereits sehr früh, d. h. im ersten Lebensjahr, verknöchern (1). Es wird angenommen, dass Änderungen der Nahrungszusammensetzung, die eine erhöhte Kaukraft erforderten, in der Evolution als Adaptation zu einer Fusion der beiden Anteile geführt haben (2, 3), um Kau- und Scherkräften besser widerstehen zu können und als Versteifung für eine schnellere Kraftübertragung, vor allem transversaler Kräfte, zu dienen (3). Die komplette Fusion der Symphyse fand entwicklungsgeschichtlich vermutlich bereits bei den frühen Antropoiden (3) und ihren Vorgängern vor über 33 bis 56 Millionen Jahren im Zeitalter des Eozäns statt (2). Die Verknöcherung ist daher abhängig von genetischen Faktoren und der Art der Nahrung und erfolgt beim Menschen in der Regel innerhalb des 1. bis 2. Lebensjahres (1, 4, 5), um die Zeit, um die sich eine Okklusion entwickelt (3).

Erhöhte inzisale, okklusale (2), vor allem aber die Übertragung transversaler Kräfte und Spannungen während des Kauvorgangs, begünstigen eine Kalzifizierung bzw. Ossifikation und Fusion der Symphyse (2, 3). Eine Kompression der Symphyse durch den Muskelzug des M. pterygoideus lateralis wird ebenfalls als begünstigender Einflussfaktor für diesen Prozess angenommen (2). Das symphysiale Knorpelgewebe spielt für die Breitenentwicklung des Unterkiefers beim Menschen daher praktisch keine Rolle, da dort bis zur Fusion der beiden Mandibulahälften wenig Wachstum stattfindet, und es auch im Anschluss, abgesehen von einer geringen Apposition an der äußeren Knochenoberfläche, zu keiner Breitenzunahme im Bereich der Symphyse mehr kommt (5).

Die intercanine Distanz und Zahnbogenbreite nimmt im Unterkiefer, im Gegensatz zum Oberkiefer, bei dem es bis zum Durchbruch der permanenten Eckzähne noch zu einer transversalen Zunahme kommt, nur bis zum Durchbruch der seitlichen permanenten Inzisivi zu und bleibt dann konstant (6), so dass die knöcherne Breite des Unterkiefers relativ früh vorgegeben ist, und die Raumverhältnisse für die Unterkieferfrontzähne im Allgemeinen sehr knapp sind (7). Die größte transversale Entwicklung des Unterkiefers ist posterior durch die divergierende Wachstumsrichtung der aufsteigenden Unterkieferäste am ausgeprägtesten (8). Anterior nimmt der Unterkiefer also nicht an Breite zu, nur distal kommt es mit zunehmendem Alter zu einer Expansion im Sinne des V-Prinzips, durch die Verlängerung der Rami (5, 8). Während der ersten Wechselgebissperiode kommt es daher nur zu einer geringen Breitenzunahme der Kieferbögen von 2 bis 3 mm, die Länge nimmt hingegen um etwa 10 bis 12 mm zu und bildet das Molarenfeld für den Durchbruch der 2. Molaren (9). Auch im folgenden Verlauf ist die Breitenzunahme des Unterkiefers irrelevant, während durch eine weitere Längenzunahme Platz für den Durchbruch der Weisheitszähne geschaffen wird (9). Bei der Transformation des Unterkiefers zu seiner endgültigen Form kommt es daher vor allem zu einer Längenzunahme des Ramus bei gleichzeitigem Zurückbleiben seiner Breitenzunahme, einer Abnahme des Kieferwinkels (10) und gleichzeitiger Vergrößerung und Ausformung des Kiefergelenks (9). Der Breitenentwicklung der Mandibula sind daher insgesamt enge Grenzen gesetzt (9) und die knöcherne Breite des Unterkiefers durch konventionelle kieferorthopädische Maßnahmen nur bedingt beeinflussbar.

Bei ausgeprägtem Platzmangel stellt die Symphysendistraktion ein alternatives Verfahren zur Extraktionstherapie permanenter Zähne zwecks Platzbeschaffung dar. Erstmals beschrieben wurde das Verfahren der Distraktion 1905 von Codivilla, der auf diese Weise lange Röhrenknochen verlängerte (11). Guerrero et al. 1997 (12) war der Erste, der diese Methode auf den Symphysenbereich übertrug und erfolgreich Platz im zahntragenden Kieferabschnitt schaffen konnte. Im Vergleich zu anderen chirurgischen Verfahren ist diese Vorgehensweise jedoch noch relativ neu, und aufgrund der Tatsache, dass es mit vielen Nebenwirkungen behaftet ist und die Bauweise konventioneller Apparaturen für den chirurgischen Anwendungsbedarf stark verbesserungsfähig ist, ist diese Methode nicht sehr weit verbreitet und findet bis heute keine standardmäßige Anwendung. Entsprechend konnte in einer aktuellen Studie zu Kriterien zum Extraktionsentscheid anhand der Befragung von 28 Kieferorthopädien mit mehr als 15 Jahren Berufserfahrung gezeigt werden, dass neben dem Profilverlauf das Ausmaß des Engstandes zweithäufigstes Kriterium für den Entscheid für eine Extraktion war, und im Falle einer Extraktionstherapie zu 66 % vier Prämolaren bzw. zu 28 % ein unterer Frontzahn für die Extraktion vorgesehen wurde, um ausreichenden Platz im Zahnbogen und eine Verbesserung der Okklusionsverhältnisse zu schaffen. Nur in 11 % der Fälle entschied man sich in der genannten Studie alternativ für eine Distraktion zum Platzgewinn (13).

Unter den bekannten Distraktoren unterscheidet man grundsätzlich zahngetragene, d. h. dentale Distraktoren, und knochengetragene, d. h. skelettale Distraktoren. Da bei dental getragenen Distraktoren unerwünschte Zahnbewegungen auftreten können (14), werden vermehrt skelettal getragene Distraktoren bevorzugt, die beidseits lateral der Unterkiefersymphyse fixiert werden und einen transgingivalen Anteil mit Aktivator-Schraube im Bereich des Vestibulums besitzen. Alle derzeit bekannten skelettalen Systeme stellen eine Ein-Stück-Bauweise eines konventionell hergestellten Gerätes dar.

Die Form der Unterkiefersymphyse und des Unterkieferkorpus unterliegen einer hohen interindividuellen Variabilität (15). Daher erfordern die konventionellen Systeme immer eine Anpassung an die jeweilige Anatomie des Patienten, die neben operativen Nachteilen (Zeitaufwand, Versetzungsgefahr anatomischer Strukturen) zu einer relativen Passungenauigkeit, schlechten Platzierung des Schraubapparates, Gewebeirritationen und Folgeschäden wie u. a. Karies oder Druckstellen führen können.

Der aufgrund entstehender klinischer Symptome wie Ulzerationen von Lippen- und/oder Weichgeweben bestehende dringende klinische Behandlungsbedarf führt oft zu einer Abtrennung des Schraubapparates, obwohl die Kallusheilung (Mineralisation des sich neu bildenden Knochens - mindestens drei Monate nach aktiver Distraktion gefordert) noch nicht abgeschlossen ist. Die Abtrennung des Schraubapparates muss aufgrund der skelettalen Lage im Munde des Patienten mit Fräsen erfolgen, kann zu weiteren Verletzungen führen und unerwünschte Kräfte auf den Kallus und sich gerade neuformierenden Knochen ausüben. Ferner sind die Distraktionsbereiche (rechte und linke Unterkieferspange) während der Kallusheilung im Folgenden dann nicht mehr gegeneinander stabilisiert. Zum anderen weist der Übergangsbereich zwischen skelettaler Fixationsmöglichkeit und transgingivalem Anteil mit Schraubapparat eine erhöhte Frakturanfälligkeit auf, gerade wenn es sich um manuell angepasste Systeme handelt. Dies ist auch der Bereich, in dem später die Trennungslinie verläuft, wenn dies die klinische Situation erforderlich macht.

Für die oben aufgeführten Probleme gibt es derzeit keine zufriedenstellenden Lösungen. Alle bekannten auf dem Markt erhältlichen Distraktionssysteme beruhen auf einem unteilbaren Bauteil, das nur durch nachträgliche Manipulation (z. B. Biegen oder Abfräsen des Schraubapparates im Mund des Patienten) den klinischen Erfordernissen angepasst werden kann, z. B. bei dringendem Handlungsbedarf bei Ulzeration.

Ein einteiliger Distraktor weist einen voluminösen Schraubapparat auf. Die Verwendung eines solchen Schraubapparates über längere Zeit (Konsolidierung) führt zu Druckstellen und Ulzerationen. Er ist außerdem mit einem erschwerten Zugang zur Reinigung für den Patienten, Speiseimpaktionen, Gewebeirritationen, Gingivitis und einem erhöhten Kariesrisiko verbunden. Die Abtrennung des Schraubapparates erfolgt mit einer Fräse im Mund durch den Chirurgen mit Gefahr der Gewebeverletzung, unerwünschten Kräften auf den Kallus und/oder auf den noch nicht ausgereiften, neu mineralisierten Knochen. Nachteilig ist auch die Instabilität und Störung der Kallusausheilung und Mineralisation durch fehlende Stabilisierung der rechten und linken distrahierten Unterkieferseite bei Abtrennung des Schraubapparates.

Aufgabe der Erfindung ist es, die Nachteile nach dem Stand der Technik zu beseitigen. Es soll insbesondere ein Distraktionssystem angegeben werden, das eine leichte Montage und Demontierbarkeit aufweist und eine Verbesserung des Patienten- und Behandlerkomforts sowie des Heilungsprozesses bewirkt. Es soll ferner ein Distraktionsset zur Herstellung einer Distraktionseinrichtung und zur Herstellung einer Konsolidierungseinrichtung angegeben werden.

Diese Aufgabe wird durch die Merkmale der Ansprüche 1 und 11 gelöst. Zweckmäßige Ausgestaltungen der Erfindungen ergeben sich aus den Merkmalen der Unteransprüche.

Nach Maßgabe der Erfindung ist ein Distraktionssystem zur Erweiterung eines Kiefers vorgesehen. Das Distraktionssystem weist ein erstes Fixierungselement zur Befestigung am Kiefer sowie ein zweites Fixierungselement zur Befestigung am Kiefer auf. Es weist ferner ein erstes Brückenelement zur lösbaren Befestigung an dem ersten Fixierungselement, ein zweites Brückenelement zur lösbaren Befestigung an dem zweiten Fixierungselement und einen Distraktionszylinder zum Verändern des Abstandes zwischen dem ersten Brückenelement und dem zweiten Brückenelement auf. Außerdem weist das Distraktionssystem einen Konsolidierungssteg zur lösbaren Befestigung an dem ersten Fixierungselement und dem zweiten Fixierungselement unter Ersatz des ersten Brückenelements, des zweiten Brückenelements und des Distraktionszylinders auf. An den beiden Fixierungselementen sind entweder die beiden Brückenelemente oder der Konsolidierungssteg befestigt.

Das erfindungsgemäße Distraktionssystem ist mehrteilig. Es besteht zumindest aus folgenden Teilen: erstes Fixierungselement, zweites Fixierungselement, erstes Brückenelement, zweites Brückenelement, Distraktionszylinder und Konsolidierungssteg. Das Distraktionssystem kann weitere Teile aufweisen. Das Distraktionssystem ist in seine Teile teilbar. Das erfindungsgemäße Distraktionssystem kann zu einer Distraktionseinrichtung oder einer Konsolidierungseinrichtung zusammengesetzt werden. Das erfindungsgemäße Distraktionssystem kann somit zur Herstellung einer Distraktionseinrichtung und daran anschließend einer Konsolidierungseinrichtung verwendet werden.

Die Distraktionseinrichtung weist zumindest das erste Fixierungselement, das zweite Fixierungselement, das erste Brückenelement, das zweite Brückenelement und den Distraktionszylinder auf. Die Distraktionseinrichtung kann weitere Teile aufweisen, der Konsolidierungssteg ist jedoch kein Teil der Distraktionseinrichtung. Die Distraktionseinrichtung wird in der Distraktionsphase zur Distraktion verwendet.

Die Konsolidierungseinrichtung weist zumindest das erste Fixierungselement, das zweite Fixierungselement und den Konsolidierungssteg auf. Die Konsolidierungseinrichtung kann weitere Teile aufweisen, das erste Brückenelement, das zweite Brückenelement und der Distraktionszylinder sind jedoch kein Teil der Konsolidierungseinrichtung. Die Konsolidierungseinrichtung wird in der Konsolidierungsphase zur Konsolidierung der mittels der Distraktionseinrichtung bewirkten Distraktion verwendet. Unter Konsolidierung wird insbesondere eine Heilung des Kallus verstanden. Dadurch bleibt der in der Distraktionsphase durch die Distraktion erhaltene Abstand dauerhaft erhalten.

Die Mehrteiligkeit des erfindungsgemäßen Distraktionssystems ermöglicht eine apparative Abstimmung seiner Teile auf den jeweiligen klinischen Behandlungsabschnitt, so dass in jeder Behandlungsphase ein optimaler Komfort für den Patienten und Behandler umgesetzt werden kann. Teile des erfindungsgemäßen Distraktionssystems oder das erfindungsgemäße Distraktionssystem können für den Patienten individuell digital geplant werden. Dies betrifft auch die Vermessungen des Kiefers zur Bestimmung der Distraktionsstrecke für die spätere Eingliederung des Konsolidierungsstegs. Es ist insbesondere vorteilhaft, wenn die knochengetragenen Anteile des Distraktionssystems individuell an den Patienten angepasst sind. Zu den knochengetragenen Anteilen des Distraktionssystems können das erste und das zweite Fixierungselement gehören. Das Distraktionssystem ist demnach individuell für einen speziellen Patienten passend. Das erste Brückenelement, das zweite Brückenelement und der Distraktionszylinder können dabei aus vorgefertigten Teilen bestehen, ebenso der Konsolidierungssteg. Es ist jedoch bevorzugt, dass auch diese Teile des erfindungsgemäßen Distraktionssystems individuell an den Patienten angepasst sind.

Unter einer Distraktion wird die Vergrößerung des Abstandes zwischen zwei Knochensegmenten des Kiefers verstanden. Das erfindungsgemäße Distraktionssystem ist insbesondere zur transversalen Erweiterung eines Kiefers geeignet. Bei dem Kiefer kann es sich um den Unterkiefer oder den Oberkiefer handeln. Vorzugsweise wird das erfindungsgemäße Distraktionssystem zur Erweiterung eines Unterkiefers, besonders bevorzugt zur transversalen Erweiterung eines Unterkiefers verwendet. Der Kiefer ist vorzugsweise ein menschlicher Kiefer. Das erfindungsgemäße Distraktionssystem kann zur Herstellung einer Distraktionseinrichtung für den Unterkiefer eingesetzt werden. In diesem Fall ist die Distraktionseinrichtung ein Unterkieferdistraktor. Die Distraktionseinrichtung kann ein Symphysendistraktor sein. Das erfindungsgemäße Distraktionssystem kann zur Herstellung einer skelettal getragenen Distraktionseinrichtung verwendet werden.

Das erfindungsgemäße Distraktionssystem ermöglicht es, den Schraubapparat zu ersetzen, sobald der erforderliche Abstand am Kiefer erreicht worden ist. Bestandteile des Schraubapparates des erfindungsgemäßen Distraktionssystems sind das erste Brückenelement, das zweite Brückenelement und der Distraktionszylinder. Zum Schraubapparat können weitere Teile des erfindungsgemäßen Distraktionssystems, die sich in Kontakt mit dem ersten Brückenelement und/oder dem zweiten Brückenelement befinden, mit Ausnahme des ersten Fixierungselements und des zweiten Fixierungselements, gehören. Der Konsolidierungssteg ist nicht Teil des Schraubapparates.

Der erforderliche Abstand am Kiefer wird in der Distraktionsphase hergestellt, indem das erste Fixierungselement an einem ersten Knochensegment des Kiefers und das zweite Fixierungselement an einem anderen Knochensegment des Kiefers befestigt und der Abstand zwischen den beiden Fixierungselementen mittels des Schraubapparates vergrößert wird. Zwischen den beiden Knochensegmenten kann vor dem Anbringen der beiden Fixierungselemente operativ ein Schnitt zwischen den beiden Knochensegmenten hergestellt werden. Der erforderliche Abstand am Kiefer ist erreicht, sobald die beiden Knochensegmente des Kiefers den von einem Behandler, beispielsweise einem Kieferorthopäden oder fachlich spezialisierten Zahnarzt vorgegebenen Abstand erreicht haben. Sobald der erforderliche Abstand zwischen den beiden Knochensegmenten erreicht ist, kann der Schraubapparat, einschließlich des ersten Brückenelements und des zweiten Brückenelements, von dem ersten Fixierungselement und dem zweiten Fixierungselement abgenommen und durch den Konsolidierungssteg ersetzt werden. Mit dem Erreichen des erforderlichen Abstands am Kiefer endet die Distraktionsphase. Anschließend kann die Konsolidierungsphase beginnen. Dazu wird der Schraubapparat von den beiden Fixierungselementen abgenommen und der Konsolidierungssteg an den beiden Fixierungselementen befestigt. Ist der erforderliche Abstand zwischen den beiden Knochensegmenten des Kiefers erreicht, wird das Verstellen des Distraktionszylinders am Schraubapparat beendet. Es ist aber nicht zwingend erforderlich, unmittelbar am Ende der Distraktionsphase den Schraubapparat durch den Konsolidierungssteg zu ersetzen, auch wenn das möglich ist. Es spricht jedoch nichts dagegen, dies später zu tun. In diesem Falle muss der Schraubapparat arretiert werden, beispielsweise mittels eines Stoppelementes, damit sich der Distraktionszylinder nicht zurückdrehen kann. Die Konsolidierung ist ein biologischer Prozess. Sie beginnt in der Regel, sobald die Distraktionsvorrichtung mehr als ca. zwei Tage nicht gestellt wurde. Es ist vorteilhaft, den Schraubapparat möglichst frühzeitig nach Ende der Distraktionsphase zu ersetzen, um den Komfort für den Patienten zu verbessern.

Erfindungsgemäß kann somit vorgesehen sein, dass an den beiden Fixierungselementen, die am Kiefer des Patienten befestigt sind, entweder der Schraubapparat oder der Konsolidierungssteg befestigt sind. Um den Schraubapparat durch den Konsolidierungssteg ersetzen zu können, ist vorgesehen, dass die beiden Brückenelemente lösbar an den beiden Fixierungselementen befestigt sind. Durch ein frühzeitiges Ersetzen des Schraubapparates durch den Konsolidierungssteg kann eine Irritation von Weichgewebe, die durch den voluminösen Anteil des Schraubapparates im Vestibulum hervorgerufen wird, reduziert werden.

Das erfindungsgemäße Distraktionssystem ermöglicht eine leichte Montage und Demontage seiner Teile, insbesondere der beiden Brückenelemente an den beiden Fixierungselementen und des Konsolidierungssteges an den beiden Fixierungselementen. Die leichte Montage und Demontage kann durch eine hohe Präzision und Passgenauigkeit der Teile des erfindungsgemäßen Distraktionssystems weiter verbessert werden. Insbesondere die Demontage des Schraubapparates kann vorgenommen werden, ohne unerwünschte Kräfte auf den mineralisierenden Kallus auszuüben, der zwischen den beiden Knochensegmenten des Kiefers nach dem Erreichen des erforderlichen Abstands ausgebildet wird. Das erfindungsgemäß vorgesehene Verbindungsdesign verhindert, dass unerwünschte Kräfte auf den mineralisierenden Kallus ausgeübt werden. Der Einsatz des erfindungsgemäßen Distraktionssystems ermöglich eine ungestörte Kallusheilung durch Stabilisierung der beiden Knochensegmente, an denen die Konsolidierungseinrichtung befestigt ist, während der Konsolidierungsphase. Das heißt mit anderen Worten, dass das rechte Knochensegment gegen das linke Knochensegment, die vor Beginn der Distraktion operativ voneinander getrennt und distrahiert worden sind, mittels der erfindungsgemäßen Konsolidierungseinrichtung während der Konsolidierungsphase stabilisiert ist.

Die Verwendung des erfindungsgemäßen Distraktionssystems ist mit einem erhöhten Komfort für Patient und Behandler verbunden. Es reduziert die Gefahr von Verletzungen, die beispielsweise mit dem Abfräsen des Schraubapparates bei Distraktoren des Standes der Technik verbunden sind.

Es kann vorgesehen sein, dass die beiden Brückenelemente jeweils einen Brückenkörper mit einer quaderförmigen, beispielsweise einer plattenförmigen Grundform aufweisen. Der Ausdruck "Grundform" soll verdeutlichen, dass die Gestalt des jeweiligen Brückenkörpers von der geometrischen Idealform abweichen kann. Der Brückenkörper besitzt vorzugsweise eine Unterseite, die einem der Fixierungselemente zugewandt ist, wenn das Brückenelement an dem Fixierungselement befestigt ist. Der Brückenkörper besitzt ferner eine Oberseite, die der Unterseite gegenüberliegt und dem Fixierungselement abgewandt ist, wenn das Brückenelement an dem Fixierungselement befestigt ist. Die Oberseite und die Unterseite des Brückenkörpers sind durch Seitenflächen miteinander verbunden, wobei zwei sich gegenüberliegende Seitenflächen die Stirnseiten des Brückenkörpers bilden. Der Brückenkörper ist vorzugsweise ein steifer Körper. Er kann beispielsweise aus einem Metall, wie z. B. Titan, einer Metalllegierung, wie z. B. einer Titanlegierung oder Stahl, oder Keramik bestehen.

In dem Brückenkörper kann eine Gewindebohrung ausgebildet sein. Die Gewindebohrung ermöglicht die Einführung des Distraktionszylinders in den Brückenkörper und damit in das Brückenelement. Die Gewindebohrung kann sich von einer Stirnseite des Brückenkörpers zu dessen gegenüberliegender Stirnseite erstrecken. Sie kann eine Durchgangsbohrung sein. Es kann somit vorgesehen sein, dass das erste Brückenelement und das zweite Brückenelement jeweils einen Brückenkörper mit einer Gewindebohrung zur Einführung des Distraktionszylinders aufweisen.

Der Distraktionszylinder weist vorzugsweise eine zylindrische Grundform auf. Der Ausdruck "Grundform" soll verdeutlichen, dass die Gestalt des Distraktionszylinders von der geometrischen Idealform abweichen kann. Er kann zwei gegenläufige Au-βengewinde aufweisen. Die Außengewinde sind zum Eingriff in Innengewinde bestimmt, die in der Gewindebohrung des ersten Brückenelements und in der Gewindebohrung des zweiten Brückenelementes ausgebildet sind. Dabei greift eines der beiden Außengewinde in das Innengewinde ein, das in dem Brückenkörper des ersten Brückenelementes ausgebildet ist, während das andere Außengewinde in das Innengewinde eingreift, das in dem Brückenkörper des zweiten Brückenelementes ausgebildet ist. Durch Drehen des Distraktionszylinders um seine Längsachse kann somit der Abstand zwischen den beiden Brückenelementen verändert werden. Das Distraktionssystem kann ein Stellelement aufweisen, das auf dem Distraktionszylinder sitzt. Das Stellelement ist ein Teil des Distraktionssystems, das zum Schraubapparat gehört. Das Stellelement kann einen Ring aufweisen, der drehfest an dem Distraktionszylinder befestigt ist. An der Außenseite des Ringes können Ausnehmungen ausgebildet sein, in die der Behandler zum Verdrehen des Ringes und damit des Distraktionszylinders mittels eines Stiftes eingreifen kann. Nach dem Verdrehen des Ringes kann ein Stoppelement in die Ausnehmung eingesetzt werden, die eine weitere Drehung des Distraktionszylinders blockiert. Zum Verdrehen des Ringes wird das Stoppelement wieder aus der Ausnehmung entnommen. Der Distraktionszylinder ist vorzugsweise ein steifer Körper. Er kann beispielsweise aus einem Metall, wie z. B. Titan, einer Metalllegierung, wie z. B. einer Titanlegierung oder Stahl, oder Keramik bestehen. Ebenso ist das Stellelement vorzugsweise ein steifer Körper. Es kann beispielsweise aus einem Metall, wie z. B. Titan, einer Metalllegierung, wie z. B. einer Titanlegierung oder Stahl, oder Keramik bestehen. Das Stellelement ist Bestandteil des Schraubapparates.

Es kann vorgesehen sein, dass das erfindungsgemäße Distraktionssystem zumindest einen Führungsstift zum Führen des ersten Brückenelementes und des zweiten Brückenelementes aufweist. Vorzugsweise weist das erfindungsgemäße Distraktionssystem zwei Führungsstifte, besonders bevorzugt genau zwei Führungsstifte auf. Mittels der Führungsstifte soll ein Verkanten der beiden Brückenelemente während der Distraktion verhindert werden. Das erste Brückenelement und das zweite Brückenelement können jeweils eine Führungsbohrung zur Aufnahme eines Führungsstiftes aufweisen. Die Zahl der Führungsbohrungen, die ein Brückenelement aufweist, entspricht dabei der Zahl der Führungsstifte. Die Führungsbohrungen erstrecken sich vorzugsweise von einer der Stirnseiten des Brückenkörpers zu dessen gegenüberliegender Stirnseite. Sie können Durchgangsbohrungen sein. Die Längsachsen der Führungsbohrungen verlaufen in dem Brückenkörper der beiden Brückenelemente vorzugsweise parallel zur Längsachse der Gewindebohrung. Vorzugsweise sind die Führungsbohrungen von der Gewindebohrung beabstandet. Die Längsachsen der Führungsbohrungen und die Längsachse der Gewindebohrung liegen vorzugsweise in einer Ebene. Diese Ebene liegt vorzugsweise parallel zur Unterseite und/oder Oberseite des Brückenkörpers. Vorzugsweise liegt die Längsachse der Gewindebohrung zwischen den Längsachsen der beiden Führungsbohrungen. Ist der Schraubapparat an den beiden Fixierungselementen montiert, so verlaufen die Längsachsen der beiden Führungsstifte vorzugsweise parallel zueinander und/oder parallel zur Längsachse des Distraktionszylinders. Der Distraktionszylinder ist vorzugsweise zwischen den beiden Führungsstiften angeordnet. Dabei ist er von den Führungsstiften beabstandet. Die Führungsstifte sind Bestandteile des Schraubapparates. Die Führungsstifte sind Teile des Distraktionssystems. Als Teile des Schraubapparates gehören sie zur Distraktionseinrichtung. Sie werden mit dem Schraubapparat nach der Distraktionsphase entfernt. Die Konsolidierungseinrichtung weist keine Führungsstege auf.

Es kann vorgesehen sein, dass das erste Fixierungselement und das zweite Fixierungselement jeweils einen ersten Abschnitt aufweisen, in dem zumindest eine Öffnung zur Durchführung eines Befestigungsmittels ausgebildet ist. Mittels der Öffnungen und Befestigungsmittel kann eine stabile Befestigung der beiden Fixierungselemente am Kiefer erreicht werden. Die stabile Befestigung der beiden Fixierungselemente am Kiefer ist vorzugsweise verwindungsstabil. Bei dem Befestigungsmittel kann es sich beispielsweise um eine Schraube handeln. Mittels des Befestigungsmittels wird das Fixierungselement, durch dessen Öffnung das Befestigungsmittel geführt ist, an einem Knochensegment des Kiefers befestigt. Dabei wird das erste Fixierungselement über seinen ersten Abschnitt an einem ersten Knochensegment des Kiefers und das zweite Fixierungselement über seinen ersten Abschnitt an einem zweiten Knochensegment des Kiefers befestigt. Das erste Fixierungselement und das zweite Fixierungselement können jeweils einen zweiten Abschnitt zur lösbaren Befestigung entweder an einem der beiden Brückenelemente oder an dem Konsolidierungssteg aufweisen. Dabei wird das erste Fixierungselement über seinen zweiten Abschnitt an dem zweiten Brückenelement und das zweite Fixierungselement über seinen zweiten Abschnitt an dem zweiten Fixierungselement befestigt. In dem ersten Abschnitt können drei oder mehr Öffnungen zur Durchführung von Befestigungsmitteln ausgebildet sein. Die Anzahl an Öffnungen hängt vor allem von der Anatomie des Patienten und/oder Größe des Führungselementes, insbesondere seines ersten Abschnittes ab. In der Regel sind drei oder vier Öffnungen vorgesehen. Auf diese Weise kann eine stabile, insbesondere verwindungsstabile Befestigung der beiden Fixierungselemente am Kiefer vorteilhaft erreicht werden. Die Zahl der Befestigungsmittel sollte der Zahl der Öffnungen entsprechen. In einer Ausführungsform sind vier Öffnungen vorgesehen. Zum Anbringen der beiden Fixierungselemente am Kiefer können zunächst Bohrungen in den Kiefer eingebracht werden, deren Lage der Anordnung der Öffnungen in den ersten Abschnitten der beiden Fixierungselemente entspricht.

Zum Herstellen der Bohrungen im Kiefer kann eine Schablone verwendet werden. Eine solche Schablone wird auch als Operations-Guide bezeichnet. Die Verwendung einer Schablone ist allerdings nicht zwingend erforderlich. Sind die beiden Fixierungselemente individuell an den Kiefer des Patienten angepasst, so können die Bohrungen mit den Fixierungselementen gesetzt werden. Der Operations-Guide kann jedoch vor allem zur Führung der Osteotomie, d. h. des Knochenschnittes, zur Trennung der beiden Knochensegmente verwendet werden. Mit dem Operations-Guide können aber auch die Bohrungen für die Befestigung der Fixierungselemente gemacht werden, da der Operations-Guide ebenfalls nur an einer Stelle am Kiefer des Patienten passt. Der Operations-Guide ist zweckmäßigerweise an die individuelle Anatomie des Patienten, einschließlich der Stellung der Frontzähne angepasst. Er kann an den Frontzähnen und dem unteren Kieferabschnitt verwindungsstabil anliegen.

Zur Herstellung der Distraktionseinrichtung wird der Schraubapparat an den beiden Fixierungselementen befestigt. Dazu wird das erste Brückenelement an dem ersten Fixierungselement befestigt, während das zweite Brückenelement an dem zweiten Fixierungselement befestigt wird. Die Verbindung zwischen dem erste Brückenelement und dem ersten Fixierungselement ist eine lösbare Verbindung. Die Verbindung zwischen dem zweiten Brückenelement und dem zweiten Fixierungselement ist eine lösbare Verbindung. Die Verbindungen sind vorzugsweise feste Verbindungen, so dass eine Bewegung der Brückenelemente relativ zu den Fixierungselementen, an denen sie befestigt sind, nicht möglich ist.

Das erste Brückenelement und das zweite Brückenelement können jeweils eine Ausnehmung zur Aufnahme des zweiten Abschnittes von einem der beiden Fixierungselemente aufweisen. Die Ausnehmung kann derart ausgebildet sein, dass eine lösbare Verbindung zwischen dem Brückenelement und dem jeweiligen Fixierungselement herstellbar ist. Die Ausnehmung, die an dem ersten Brückenelement ausgebildet ist, dient dabei zur Aufnahme des zweiten Abschnittes des ersten Fixierungselements. Der zweite Abschnitt des ersten Fixierungselements kann formschlüssig in der Ausnehmung des ersten Brückenelementes anliegen. Die Ausnehmung, die an dem zweiten Brückenelement ausgebildet ist, dient dabei zur Aufnahme des zweiten Abschnittes des zweiten Fixierungselementes. Der zweite Abschnitt des zweiten Fixierungselements kann formschlüssig in der Ausnehmung des zweiten Brückenelementes anliegen. Vorzugsweise ist die Ausnehmung an der Unterseite des Brückenkörpers des jeweiligen Brückenelements ausgebildet. Die Ausnehmung kann so ausgebildet sein, dass der zweite Abschnitt in der Ausnehmung mit einer seiner Flächenseiten derart an dem Brückenkörper anliegt, dass seine gegenüberliegende Flächenseite fluchtend mit der Unterseite des Brückenkörpers abschließt.

Zur Herstellung der Konsolidierungseinrichtung wird der Schraubapparat an den beiden Fixierungselementen entfernt und durch den Konsolidierungssteg ersetzt. Damit wird die Distraktionseinrichtung in die Konsolidierungseinrichtung überführt. Dazu wird der Konsolidierungssteg an dem ersten Fixierungselement und an dem zweiten Fixierungselement befestigt. Die Verbindung zwischen dem Konsolidierungssteg und dem ersten Fixierungselement ist eine lösbare Verbindung. Die Verbindung zwischen dem Konsolidierungssteg und dem zweiten Fixierungselement ist eine lösbare Verbindung. Die Verbindungen sind vorzugsweise feste Verbindungen, so dass eine Bewegung des Konsolidierungssteges relativ zu den Fixierungselementen, an denen es befestigt ist, nicht möglich ist.

Der Konsolidierungssteg kann eine erste Ausnehmung zur Aufnahme des zweiten Abschnittes des ersten Fixierungselementes und eine zweite Ausnehmung zur Aufnahme des zweiten Abschnittes des zweiten Fixierungselementes aufweisen. Die erste Ausnehmung des Konsolidierungssteges kann derart ausgebildet sein, dass eine lösbare Verbindung zwischen dem Konsolidierungssteg und dem ersten Fixierungselement herstellbar ist. Die zweite Ausnehmung des Konsolidierungssteges kann derart ausgebildet sein, dass eine lösbare Verbindung zwischen dem Konsolidierungssteg und dem zweiten Fixierungselement herstellbar ist. Vorzugsweise ist die Ausnehmung an einer Flächenseite des Konsolidierungssteges ausgebildet. Diese Flächenseite ist den beiden Fixierungselementen zugewandt, wenn der Konsolidierungssteg an den Fixierungselementen montiert ist. Die erste Ausnehmung kann so ausgebildet sein, dass der zweite Abschnitt des ersten Fixierungselements in der ersten Ausnehmung mit einer seiner Flächenseiten derart an dem Konsolidierungssteg anliegt, dass seine gegenüberliegende Flächenseite fluchtend mit der Flächenseite des Konsolidierungssteges abschließt. Die zweite Ausnehmung kann so ausgebildet sein, dass der zweite Abschnitt des zweiten Fixierungselements in der zweiten Ausnehmung mit einer seiner Flächenseiten derart an dem Konsolidierungssteg anliegt, dass seine gegenüberliegende Flächenseite fluchtend mit der Flächenseite des Konsolidierungssteges abschließt.

Der Abstand zwischen der ersten Ausnehmung und der zweiten Ausnehmung des Konsolidierungssteges wird durch den Abstand bestimmt, der zwischen den Knochensegmenten eingestellt werden soll. Dieser Abstand ist bei Beginn der Distraktion bekannt. Der Konsolidierungssteg kann somit individuell für den Patienten hergestellt werden, so dass der Abstand zwischen seiner ersten Ausnehmung und seiner zweiten Ausnehmung genau dem Abstand entspricht, den die beiden zweiten Abschnitte des ersten und zweiten Fixierungselementes am Ende der Distraktionsphase aufweisen.

Das erste Fixierungselement und das zweite Fixierungselement können jeweils einen Verbindungsabschnitt aufweisen, der den ersten Abschnitt des jeweiligen Fixierungselementes mit dessen zweitem Abschnitt verbindet. Der erste Abschnitt eines Fixierungselementes ist vorzugsweise an die Anatomie des Patientenkiefers angepasst. Er ist in diesem Fall individuell gestaltet. Er kann eine plattenförmige Grundform aufweisen. Der Ausdruck "Grundform" soll verdeutlichen, dass die Gestalt des ersten Abschnittes von der geometrischen Idealform abweichen kann. Er kann gewölbt sein. Der zweite Abschnitt eines Fixierungselementes kann eine plattenförmige Grundform aufweisen. Der Ausdruck "Grundform" soll verdeutlichen, dass die Gestalt des zweiten Abschnittes von der geometrischen Idealform abweichen kann. Der Verbindungsabschnitt eines Fixierungselementes kann stegförmig ausgebildet sein. Er verbindet vorzugsweise eine Schmalseite des ersten Abschnittes mit einer Schmalseite des zweiten Abschnittes. Der Verbindungsabschnitt kann gekrümmt sein. Im Hinblick darauf, dass die ersten Abschnitte der beiden Fixierungselemente am Kiefer befestigt werden sollen, können die Fixierungselemente insgesamt auch als Pelotten bezeichnet werden. Der Verbindungsabschnitt bildet gemeinsam mit dem ersten Abschnitt einen Arm. Der Arm kann sich von dem ersten Abschnitt weg erstrecken.

Der zweite Abschnitt eines Fixierungselementes ermöglicht es, eine Verbindung zwischen dem Fixierungselement und einem Brückenelement oder eine Verbindung zwischen dem Fixierungselement und dem Konsolidierungssteg herzustellen. Beide Verbindungen sind lösbar. Nach der Befestigung der beiden Fixierungselemente am Kiefer des Patienten können zunächst die beiden Brückenelemente - und mit ihnen der Schraubapparat - an den beiden montierten Fixierungselementen befestigt werden, wodurch die Distraktionseinrichtung erhalten wird. Nachdem der erforderliche Abstand am Kiefer erreicht ist, kann der Schraubapparat - und mit ihm die beiden Brückenelemente - entfernt und durch den Konsolidierungssteg ersetzt werden, wodurch die Konsolidierungseinrichtung erhalten wird.

Es können zweite Befestigungsmittel zur lösbaren Befestigung des ersten Fixierungselements an dem ersten Brückenelement oder an dem Konsolidierungssteg und zur lösbaren Befestigung des zweiten Fixierungselements an dem zweiten Brückenelement oder an dem Konsolidierungssteg vorgesehen sein. Mittels der zweiten Befestigungsmittel können (i) die lösbare Verbindung zwischen dem ersten Fixierungselement und dem ersten Brückenelement oder dem Konsolidierungssteg und (ii) die lösbare Verbindung zwischen dem zweiten Fixierungselement und dem zweiten Brückenelement oder dem Konsolidierungssteg hergestellt werden. Es ist jedoch nicht zwingend erforderlich, die lösbaren Verbindungen mittels zweiter Befestigungsmittel herzustellen. Die lösbaren Verbindungen können auch auf anderem Wege hergestellt werden.

Bei den zweiten Befestigungsmitteln kann es sich um Schrauben handeln. Es kann vorgesehen sein, dass ein Hineindrehen der Schrauben in die Fixierungselemente die Herstellung einer Verbindung zwischen den Fixierungselementen und den Brückenelementen des Schraubapparates bewirkt, während ein Herausdrehen der Schrauben aus den Fixierungselementen eine Lösung der Verbindung zwischen den Fixierungselementen und den Brückenelementen des Schraubapparates bewirkt, wodurch ein leichteres Entfernen des Schraubapparates ermöglicht wird. Die zweiten Befestigungsmittel gehören nicht zum Schraubapparat. Mittels der zweiten Befestigungsmittel kann die lösbare Verbindung zwischen dem ersten Fixierungselement und dem ersten Brückenelement oder dem Konsolidierungssteg einerseits und zwischen dem zweiten Fixierungselement und dem zweiten Brückenelement oder dem Konsolidierungssteg anderseits hergestellt und/oder gesichert werden. Es kann vorgesehen sein, dass der Brückenkörper des Brückenelementes eine Befestigungsbohrung aufweist. Die Befestigungsbohrung kann sich von seiner Oberseite bis zu seiner Unterseite erstrecken. Eine korrespondierende Bohrung kann in dem Fixierungselement ausgebildet sein, vorzugsweise im zweiten Abschnitt des Fixierungselementes. Die korrespondiere Bohrung kann eine Durchgangsbohrung sein. Das zweite Befestigungsmittel kann durch die Befestigungsbohrung, die im Brückenkörper des Brückenelementes ausgebildet ist, und die korrespondiere Bohrung des zweiten Abschnittes des Fixierungselementes geführt werden, wodurch die lösbare Verbindung zwischen dem Brückenelement und dem Fixierungselement hergestellt und/oder gesichert wird. Dazu können die zweiten Befestigungsmittel jeweils ein Außengewinde aufweisen, die in Innengewinde eingreifen, die in der Befestigungsbohrung und der korrespondierenden Bohrung ausgebildet sind.

Der Konsolidierungssteg ist vorzugsweise ein steifer Körper. Er kann beispielsweise aus einem Metall, wie z. B. Titan, einer Metalllegierung, wie z. B. einer Titanlegierung oder Stahl, oder Keramik bestehen. Das erste und das zweite Fixierungselement sind vorzugsweise steife Körper. Sie können beispielsweise aus einem Metall, wie z. B. Titan, einer Metalllegierung, wie z. B. einer Titanlegierung oder Stahl, oder Keramik bestehen. Es kann vorgesehen sein, dass der Konsolidierungssteg zwei Befestigungsbohrungen aufweist. In diese Befestigungsbohrung greifen die zweiten Befestigungsmittel ein, wenn der Konsolidierungssteg an den beiden Fixierungselementen befestigt ist. Die Befestigungsbohrungen des Konsolidierungssteges weisen vorzugsweise jeweils ein Innengewinde auf. Die Befestigungsbohrungen des Konsolidierungssteges können Durchgangsbohrungen sein, die sich, wenn der Konsolidierungssteg mit den beiden Fixierungselementen durch lösbare Verbindungen verbunden ist, von einer Flächenseite des Konsolidierungssteges, die den beiden Fixierungselementen abgewandt ist, zu der Flächenseite des Konsolidierungssteges erstrecken, in der sich seine beiden Ausnehmungen befinden. Die zweiten Befestigungsmittel können durch die Befestigungsbohrungen, die im Konsolidierungssteg ausgebildet sind, und die korrespondieren Bohrungen der zweiten Abschnitte der Fixierungselemente geführt werden, wodurch die lösbaren Verbindungen zwischen dem Konsolidierungssteg und den Fixierungselementen hergestellt werden. Es ist jedoch nicht zwingend erforderlich, die lösbaren Verbindungen zwischen dem Konsolidierungssteg und den Fixierungselementen mittels zweiter Befestigungsmittel herzustellen. Die lösbaren Verbindungen können auch auf anderem Wege hergestellt werden.

Das erfindungsgemäße Distraktionssystem kann als teilbar angesehen werden. Der Ausdruck "teilbar" beschreibt dabei die Eigenschaft des erfindungsgemäßen Distraktionssystems, den Schraubapparat durch den Konsolidierungssteg ersetzen zu können. Das erfindungsgemäße Distraktionssystem ist teilbar, weil die Fixierungselemente lösbar mit dem Schraubapparat oder dem Konsolidierungssteg verbindbar sind und weil der Schraubapparat durch den Konsolidierungssteg ersetzbar ist. Die Teilbarkeit des erfindungsgemäßen Distraktionssystems ermöglicht eine apparative Abstimmung seiner Einzelkomponenten auf den jeweiligen klinischen Behandlungsabschnitt, so dass in jeder Behandlungsphase ein optimaler Komfort für den Patienten und Behandler umgesetzt werden kann.

Die apparative Abstimmung der Einzelkomponenten des erfindungsgemäßen Distraktionssystems wird durch eine hohe Passgenauigkeit und Präzision weiter verbessert, wenn die Einzelkomponenten unter Verwendung dreidimensionaler Aufnahmen des Kiefers, auf deren Basis ein dreidimensionales Modell des erfindungsgemäßen Distraktionssystems gefertigt wird, hergestellt werden. Durch eine Vollindividualisierung anhand einer zuvor angefertigten dreidimensionalen Aufnahme, beispielsweise durch Bildgebung oder 3D-Datenfusion unter Erhalt eines digitalen Zwillings, ist eine optimale Platzierung der Einzelkomponenten des erfindungsgemäßen Distraktionssystems möglich. Die im Zusammenhang mit dem Stand der Technik genannten klinischen Nachteile bekannter Distraktoren können damit wesentlich reduziert oder ganz behoben werden, was zu einem optimalen Behandlungsergebnis beiträgt. Die dreidimensionale Aufnahme kann mittels bildgebender Verfahren wie Computertomographie (abgekürzt "CT"), Magnetresonanztomographie oder dentaler Volumentomographie angefertigt werden, wobei die dentale Volumentomographie (abgekürzt "DVT") besonders bevorzugt ist. Weitere dreidimensionale Bildgebungsverfahren wie 3D-Scan sind ebenfalls verwendbar, beispielsweise für die Darstellung der Weichgewebe oder bessere Darstellung der Zähne und/oder des Gesichtes für die Vermessung und Planung der Distraktion und des erfindungsgemäßen Distraktionssystems. Einzelheiten zur Herstellung eines Distraktionssystems unter Anfertigung einer dreidimensionalen Aufnahme des Kiefers, dem Anfertigen eines virtuellen Modells des Kiefers unter Verwendung der dreidimensionalen Aufnahme und der Herstellung des erfindungsgemäßen Distraktionssystems anhand des virtuellen Modells können DE 10 2015 118 853 A1 entnommen werden. Die dort beschriebene Herstellung eines Distraktors kann auf die Herstellung des erfindungsgemäßen Distraktionssystems, einschließlich des Konsolidierungssteges, übertragen werden.

Durch die Teilbarkeit des erfindungsgemäßen Distraktionssystems wird ein wesentlich höherer Patientenkomfort erreicht. Der voluminöse Schraubapparat wird nur in einer kurzen Behandlungsphase, der Distraktionsphase, benötigt und kann leicht gegen einen grazileren Konsolidierungssteg ausgetauscht werden. Irritationen der Weichgewebe (Lippe, Schleimhaut) mit folgenden Entzündungen können minimiert werden, und durch die bessere Reinigungsmöglichkeit für den Patienten kommt es weniger häufig zu Speiseimpaktionen, Ulzerationen und Gingivitis. Für den Behandler ist die Handhabung durch die Demontierbarkeit des Schraubapparates und leichte Montierbarkeit des Konsolidierungssteges erleichtert. Es ist kein Abfräsen des Schraubapparates notwendig, es entstehen keine unerwünschten Zugkräfte auf den Kallus, und eine dauerhafte Stabilisierung des Kallus und eine Verbesserung des Heilungsprozesses des neu mineralisierenden Knochens wird auf diese Weise gewährleistet. Das erfindungsgemäße Distraktionssystem kann somit ein vollindividualisiertes Distraktionssystem sein. Es kann ein patientenindividuelles Distraktionssystem sein. Das erfindungsgemäße Distraktionssystem ist mehrteilig, demontierbar und teilbar.

Die Umsetzbarkeit eines gegenüber konventionell vorgefertigten bekannten Distraktoren, die aus einem einzigen Bauteil bestehen und in der Regel intraoperativ vom Chirurgen angepasst, d. h. an die Anatomie des Patientenunterkiefers angebogen werden müssen, vorteilhaften individuellen 3D-geplanten und im 3D-Druck umgesetzten Designs konnte bereits in einer Machbarkeitsstudie nachgewiesen werden. Durch das neue Design mit einer Teilungsmöglichkeit des Patienten-individualisierten skelettal getragenen Distraktionssystems zur Distraktion von Knochensegmenten eines Unterkiefers werden die technischen und klinischen Probleme der einteiligen Distraktoren eliminiert und an das klinische Anforderungsprofil angepasst. Eine Passgenauigkeit höchster Präzision mit optimaler Lagepositionierung des Schraubapparates und Austauschbarkeit des Schraubapparates gegen den Konsolidierungssteg optimiert nicht nur den klinischen Komfort für Patient und Behandler, sondern führt auch zu einer verbesserten Kallusheilung nach der initialen Phase der Distraktion. Alle derzeit bekannten Distraktoren besitzen diese Möglichkeit nicht. Eine Demontage des Schraubapparates ist spannungsfrei möglich, so dass der individuell angepasste Konsolidierungssteg in dieser Phase der Kallusheilung ebenfalls spannungsfrei eingebracht werden kann und die Kiefersegmente, die auch als Distraktionssegmente bezeichnet werden, während der Mineralisation des sich neu bildenden Knochens ausreichend stabilisiert sind. In diesem oder einem vergleichbaren Anwendungsszenario ist eine derartige Bauweise bisher nicht beschrieben oder umgesetzt worden.

Werden Teile des erfindungsgemäßen Distraktionssystems aus einer Titanlegierung gefertigt, so kann es sich bei der Titanlegierung um eine CE-zugelassene Titanlegierung handeln. Solche Titanlegierungen werden beispielsweise auch für Osteosyntheseplatten verwendet. Die aus einer Titanlegierung bestehenden Teile können beispielsweise im 3D-Titandruckverfahren hergestellt werden.

Nach Maßgabe der Erfindung ist ferner ein Distraktionsset zur Herstellung einer Distraktionseinrichtung, insbesondere einer Distraktionseinrichtung zur transversalen Erweiterung eines Kiefers, und zur Herstellung einer Konsolidierungseinrichtung vorgesehen, wobei das Distraktionsset aufweist:
- ein erstes Fixierungselement zur Befestigung am Kiefer sowie ein zweites Fixierungselement zur Befestigung am Kiefer;
- zur Herstellung der Distraktionseinrichtung ein erstes Brückenelement zur lösbaren Befestigung an dem ersten Fixierungselement, ein zweites Brückenelement zur lösbaren Befestigung an dem zweiten Fixierungselement, und einen Distraktionszylinder zum Verändern des Abstandes zwischen dem ersten Brückenelement und dem zweiten Brückenelement; und
- zur Herstellung der Konsolidierungseinrichtung einen Konsolidierungssteg zur lösbaren Befestigung an dem ersten Fixierungselement und an dem zweiten Fixierungselement.

Das erfindungsgemäße Distraktionsset kann außerdem Befestigungsmittel zur Befestigung des ersten Fixierungselementes und des zweiten Fixierungselementes am Kiefer aufweisen. Es kann darüber hinaus zweite Befestigungsmittel zur lösbaren Befestigung des ersten Fixierungselements an dem ersten Brückenelement oder an dem Konsolidierungssteg und zur lösbaren Befestigung des zweiten Fixierungselements an dem zweiten Brückenelement oder an dem Konsolidierungssteg aufweisen. Überdies kann es zumindest einen Führungsstift zum Führen des ersten Brückenelementes und des zweiten Brückenelementes aufweisen. Vorzugsweise weist das erfindungsgemäße Distraktionsset zwei Führungsstifte auf.

Weitere Einzelheiten des erfindungsgemäßen Distraktionssets sind bereits im Zusammenhang mit dem erfindungsgemäßen Distraktionssystem beschrieben worden. Auf dessen Beschreibung wird verwiesen.

Die Erfindung wird nachstehend anhand von Ausführungsbeispielen, die die Erfindung nicht einschränken sollen, unter Bezugnahme auf die Zeichnungen näher erläutert. Dabei zeigen
- Fig. 1: eine Draufsicht auf eine Distraktionseinrichtung einer Ausführungsform eines erfindungsgemäßen Distraktionssystems;

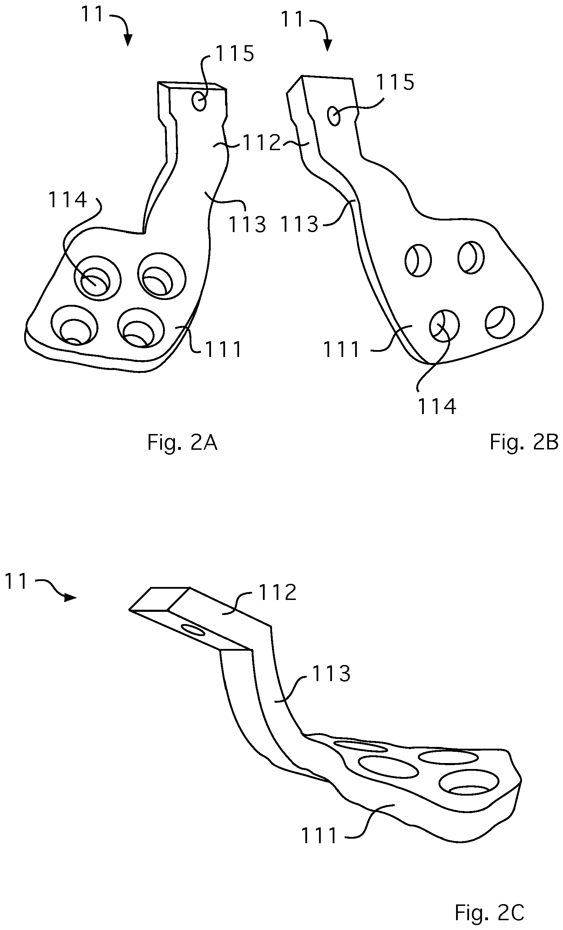

- Fig. 2: Ansichten des ersten Fixierungselementes der in Fig. 1 gezeigten Ausführungsform (Fig. 2A: Ansicht von oben; Fig. 2B: Ansicht von unten; Fig. 2C: Seitenansicht);
- Fig. 3: Ansichten des zweiten Fixierungselementes der in Fig. 1 gezeigten Ausführungsform (Fig. 3A: Ansicht von oben; Fig. 3B: Seitenansicht);
- Fig. 4: Ansichten des ersten Brückenelementes der in Fig. 1 gezeigten Ausführungsform (Fig. 4A: Ansicht von oben; Fig. 4B: Ansicht von unten);
- Fig. 5: Ansichten des zweiten Brückenelementes der in Fig. 1 gezeigten Ausführungsform (Fig. 5A: Ansicht von oben; Fig. 5B: Ansicht von unten);

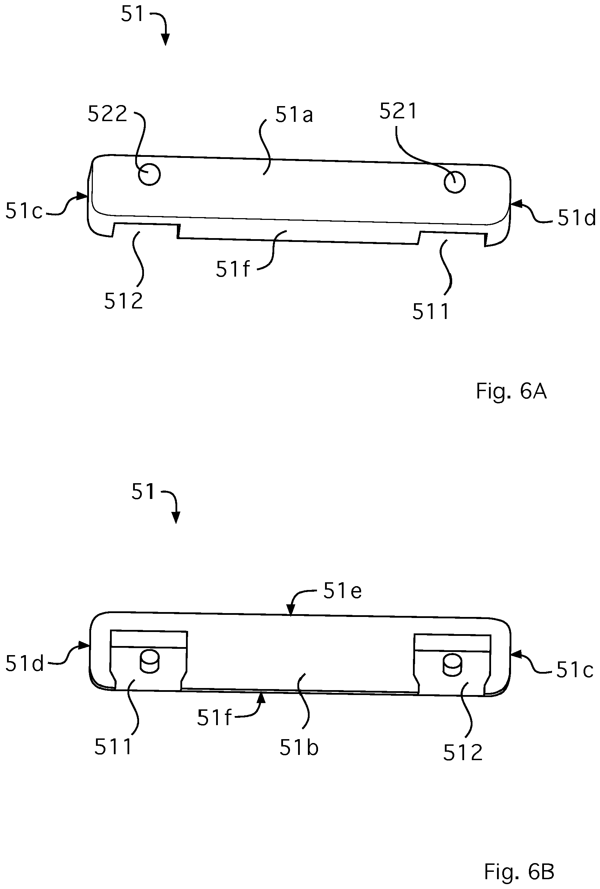

- Fig. 6: Ansichten des Konsolidierungssteges der in Fig. 1 gezeigten Ausführungsform (Fig. 6A: perspektivische Ansicht von oben; Fig. 6B: perspektivische Ansicht von unten); und
- Fig. 7: eine Draufsicht auf eine Konsolidierungseinrichtung der in Fig. 1 gezeigten Ausführungsform des erfindungsgemäßen Distraktionssystems, wobei zusätzlich der Unterkiefer eines Patienten gezeigt ist.

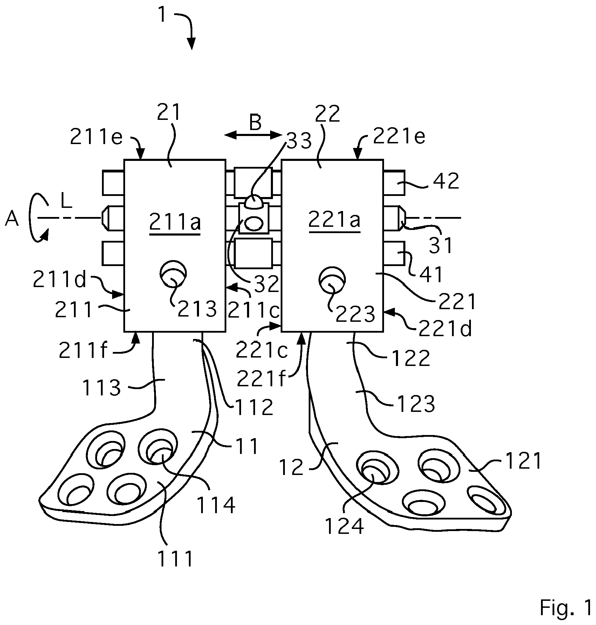

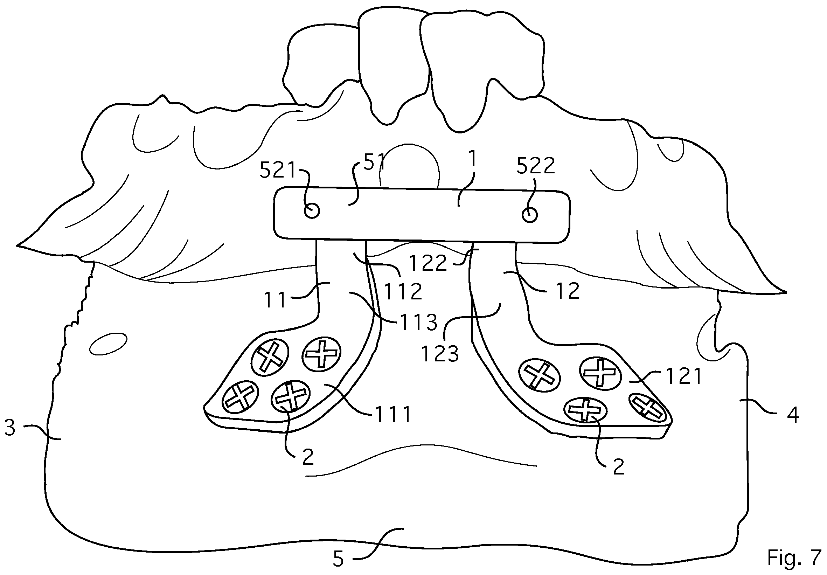

In Fig. 1 ist das erfindungsgemäße Distraktionssystem 1 in der Distraktionsphase gezeigt. Fig. 1 zeigt somit eine Distraktionseinrichtung, die mittels des erfindungsgemäßen Distraktionssystems 1 hergestellt werden kann. In Fig. 7 ist hingegen das erfindungsgemäße Distraktionssystem 1 in der Konsolidierungsphase gezeigt. Fig. 7 zeigt somit eine Konsolidierungseinrichtung, die mittels des erfindungsgemäßen Distraktionssystems 1 hergestellt werden kann.

Die in Fig. 1 gezeigte Distraktionseinrichtung weist einen Schraubapparat auf, der an dem ersten Fixierungselementen 11 und dem zweiten Fixierungselement 12 lösbar befestigt ist. Der Schraubapparat besteht aus dem ersten Brückenelement 21 und dem zweiten Brückenelement 22, dem Distraktionszylinder 31 und den beiden Führungsstiften 41, 42. Auf dem Distraktionszylinder 31 sitzt ein Stellelement 32 zum Drehen des Distraktionszylinders 31 um dessen Längsachse L (Pfeil A). In Fig. 1 ist zur Vereinfachung der Darstellung der Unterkiefer des Patienten nicht gezeigt.

Das erste Fixierungselement 11 weist einen ersten, plattenförmigen Abschnitt 111, einen zweiten, plattenförmigen Abschnitt 112 und einen stegförmigen Verbindungsabschnitt 113 auf, der den ersten Abschnitt 111 mit dem zweiten Abschnitt 112 verbindet. Der erste Abschnitt 111 ist zur Befestigung an einem Kiefersegment bestimmt. Er weist in dieser Ausführungsform dazu vier Öffnungen 114 zur Durchführung von Schrauben 2 (siehe Fig. 7) auf, die als Befestigungsmittel dienen und mit denen das erste Fixierungselement 11 an dem Kiefersegment 3 des Unterkiefers 5 eines Patienten befestigt wird. Dazu werden die Schrauben 2 durch die Öffnungen 114 geführt. Die Anzahl an Öffnungen 114 kann geringer oder größer als vier sei. Vorzugsweise sind mindestens drei Öffnungen 114 vorgesehen. Die Anzahl an Öffnungen 114 hängt vor allem von der Anatomie des Patienten und/oder Größe des ersten Führungselementes 11, insbesondere seines ersten Abschnittes 111 ab.

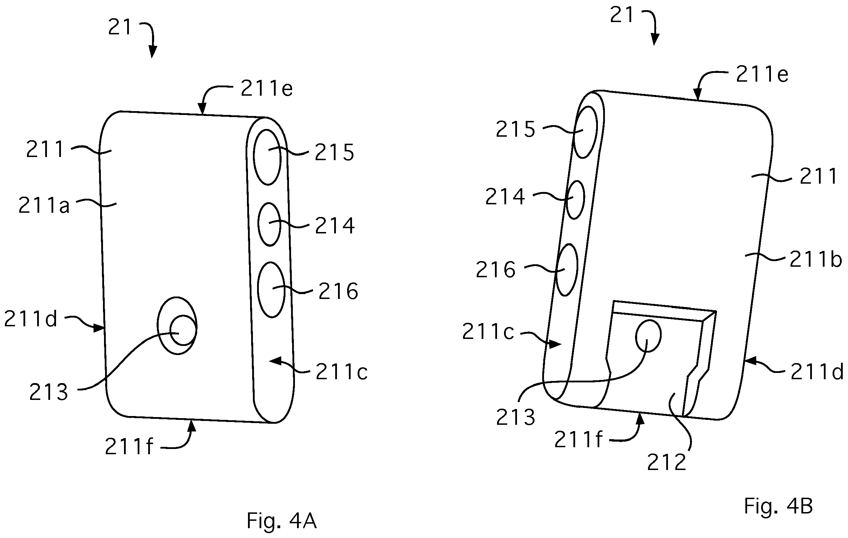

Der zweite Abschnitt 112 des ersten Fixierungselementes 11 ermöglicht eine lösbare Verbindung des ersten Fixierungselementes 11 mit dem ersten Brückenelement 21 (siehe Fig. 1) oder mit einem Konsolidierungssteg 51. Der zweite Abschnitt 112 ist so geformt, dass er in eine Ausnehmung 212 des ersten Brückenelementes 21 eingreifen kann (siehe Fig. 4B). Mittels einer Schraube (nicht gezeigt), die als zweites Befestigungsmittel dient, kann der zweite Abschnitt 112 lösbar mit dem ersten Brückenelement 21 verbunden werden. Die Verbindung kann jedoch auch auf andere Weise hergestellt werden. Das erste Fixierungselement 11 ist an die Anatomie des Patienten angepasst und somit individuell gestaltet.

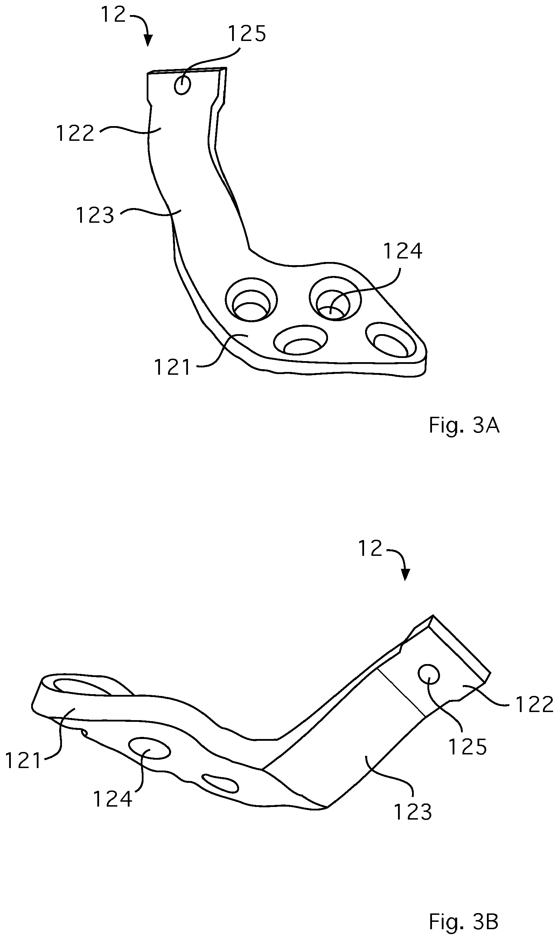

Das zweite Fixierungselement 12 (siehe Fig. 3) weist den gleichen Aufbau wie das erste Fixierungselement 11 auf, es kann allerdings im Wesentlichen spiegelsymmetrisch zum ersten Fixierungselement 11 ausgebildet sein. Die Spiegelebene erstreckt sich dabei orthogonal zur Längsachse L und orthogonal zur Papierebene von Fig. 1. Der Ausdruck "im Wesentlichen" bezieht sich auf die Anpassung des zweiten Fixierungselementes 12 an das andere Kiefersegment 4, an dem es über seinen ersten Abschnitt 121 befestigt ist, während das erste Fixierungselement 11 an dem ersten Kiefersegment 3 befestigt ist. Der zweite Abschnitt 122 des zweiten Fixierungselements 12 ist in einer Ausnehmung 222 befestigt, die an der Unterseite 221b des Brückenkörpers 221 des zweiten Brückenelementes 22 ausgebildet ist. Das zweite Fixierungselement 12 ist ebenfalls an die Anatomie des Patienten angepasst und somit individuell gestaltet.

Das zweite Fixierungselement 12 weist einen ersten, plattenförmigen Abschnitt 121, einen zweiten, plattenförmigen Abschnitt 122 und einen stegförmigen Verbindungsabschnitt 123 auf, der den ersten Abschnitt 121 mit dem zweiten Abschnitt 122 verbindet. Der erste Abschnitt 121 ist zur Befestigung an einem Kiefersegment 4 bestimmt, und zwar an einem anderen Kiefersegment als der erste Abschnitt 111 des ersten Fixierungselements 11. Der erste Abschnitt 121 weist in dieser Ausführungsform dazu vier Öffnungen 124 zur Durchführung von Schrauben 2 auf, die als Befestigungsmittel dienen und mit denen das zweite Fixierungselement 12 an dem Kiefersegment befestigt wird. Dazu werden die Schrauben durch die Öffnungen 124 geführt. Die Anzahl an Öffnungen 124 hängt vor allem von der Anatomie des Patienten und/oder Größe des zweiten Führungselementes 12, insbesondere seines ersten Abschnittes 121 ab.

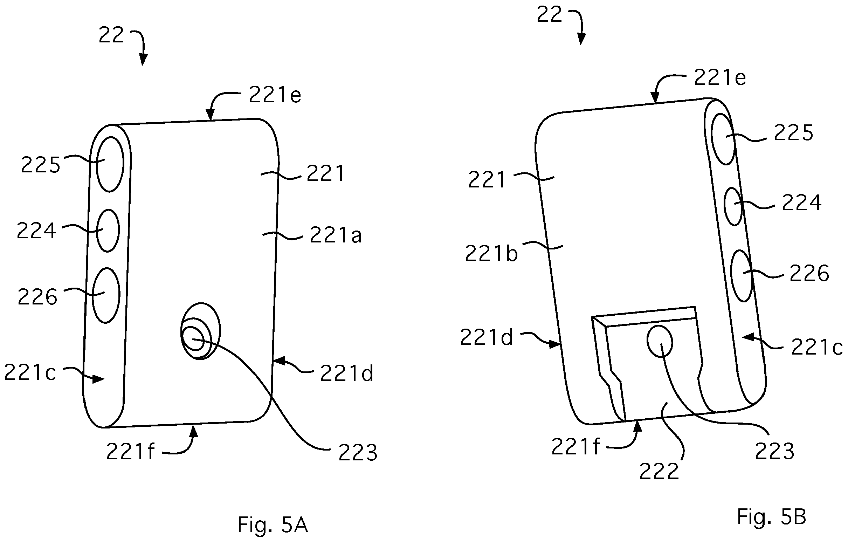

Der zweite Abschnitt 122 des zweiten Fixierungselementes 12 ermöglicht eine lösbare Verbindung des zweiten Fixierungselementes 12 mit dem zweiten Brückenelement 22 (siehe Fig. 1) oder mit dem Konsolidierungssteg 51. Der zweite Abschnitt 122 ist so geformt, dass er in eine Ausnehmung 222 des zweiten Brückenelementes 22 eingreifen kann (siehe Fig. 5B). Mittels einer Schraube (nicht gezeigt), die als zweites Befestigungsmittel dient, kann der zweite Abschnitt 122 lösbar mit dem zweiten Brückenelement 22 verbunden werden. Die Verbindung kann jedoch auch auf andere Weise hergestellt werden.

Das erste Brückenelement 21 weist einen Brückenkörper 211 auf. Der Brückenkörper 211 besitzt eine quaderförmige Grundform mit einer Oberseite 211a und einer Unterseite 211b, die der Oberseite 211a gegenüberliegt. Bei der Distraktionseinrichtung (siehe Fig. 1) ist die Unterseite 211b dem zweiten Abschnitt 112 des ersten Fixierungselementes 11 zugewandt. Bei der Distraktionseinrichtung sind sowohl das erste Brückenelement 21 an dem ersten Fixierungselement 11 als auch das zweite Brückenelement 22 an dem zweiten Fixierungselement 12 befestigt. Die Oberseite 211a und die Unterseite 211b des ersten Brückenkörpers 211 sind durch Seitenflächen miteinander verbunden, wobei zwei sich gegenüberliegende Seitenflächen die Stirnseiten 211c, 211d des Brückenkörpers 211 bilden und die verbliebenen sich gegenüberliegenden Seitenflächen die Schmalseiten 211e, 211f des Brückenkörpers 211 bilden. An der Unterseite 211b des Brückenkörpers 211 ist die Ausnehmung 212 zur Aufnahme des zweiten Abschnittes 112 des ersten Fixierungselementes 11 ausgebildet. Die Ausnehmung 212 erstreckt sich dabei bis zur Schmalseite 211f des Brückenkörpers 211. An dieser Schmalseite 211f ist die Ausnehmung 212 offen, um ein Einbringen des zweiten Abschnittes 112 des ersten Fixierungselementes 11 unter Herstellung einer lösbaren Verbindung zu ermöglichen.

Das erste Brückenelement 21 weist eine Befestigungsbohrung 213 auf, die sich von seiner Oberseite 211a bis zu seiner Unterseite 211b erstreckt. Sie endet dabei an der Ausnehmung 212. Durch die Befestigungsbohrung 213 und eine korrespondierende Bohrung 115, die im zweiten Abschnitt 112 des ersten Fixierungselementes 11 ausgebildet ist, ist eine Schraube als zweites Befestigungsmittel geführt, mit deren Hilfe das erste Brückenelement 21 mit dem ersten Fixierungselement 11 lösbar verbunden ist. Die Verwendung einer Schraube erleichtert das Lösen der Verbindung. Dazu weisen die Befestigungsbohrung 213 und die korrespondierende Bohrung 115 jeweils ein Innengewinde (nicht gezeigt) auf. Die Verbindung kann jedoch auch auf andere Weise hergestellt werden.

Das erste Brückenelement 21 weist eine Gewindebohrung 214 auf, die sich von der ersten Stirnseite 211c zu der zweiten Stirnseite 211d des Brückenkörper 211 erstreckt. Die Gewindebohrung 214 weist ein Innengewinde (nicht gezeigt) auf. Durch die Gewindebohrung ist der Distraktionszylinder 31 geführt, der ein Außengewinde (nicht gezeigt) aufweist, das in das Innengewinde der Gewindebohrung 214 eingreift. Das erste Brückenelemente 21 weist ferner zwei Führungsbohrungen 215, 216 auf, die sich ebenfalls von der ersten Stirnseite 211c zu der zweiten Stirnseite 211d des Brückenkörpers 211 erstrecken. Die Längsachsen der beiden Führungsbohrungen 215, 216 liegen parallel zur Längsachse der Gewindebohrung 214. Die beiden Führungsbohrungen 215, 216 sind von der Gewindebohrung 214 beabstandet. Die Längsachse der Gewindebohrung 214 liegt auf der Längsachse des Distraktionszylinders 31, wenn der Distraktionszylinder 31 durch die Gewindebohrung 214 geführt ist. Durch die Führungsbohrung 215 ist der Führungsstift 41, durch die Führungsbohrung 216 ist der Führungsstift 42 geführt. Weder die Führungsbohrungen 215, 216 noch die Führungsstifte 41, 42 weisen Gewinde auf. Die Funktion der Führungsstifte 41, 42 beschränkt sich auf die Führung der beiden Brückenelemente 21, 22 während der Distraktion.

Das zweite Brückenelement 22 (siehe Fig. 5) hat den gleichen Aufbau wie das erste Brückenelement 21, es kann allerdings spiegelsymmetrisch zum ersten Brückenelement 21 ausgebildet sein. Die Spiegelebene erstreckt sich dabei orthogonal zur Längsachse L und orthogonal zur Papierebene von Fig. 1. Das Innengewinde, das in der Gewindebohrung 224 des zweiten Brückenelements 22 ausgebildet ist, ist gegenläufig zum Innengewinde, das in der Gewindebohrung 214 des ersten Brückenelements 21 ausgebildet ist.

Das zweite Brückenelement 22 weist einen Brückenkörper 221 auf. Der Brückenkörper 221 besitzt eine quaderförmige Grundform mit einer Oberseite 221a und einer Unterseite 221b, die der Oberseite 221a gegenüberliegt. Bei der Distraktionseinrichtung (siehe Fig. 1) ist die Unterseite 221b dem zweiten Abschnitt 122 des zweiten Fixierungselementes 12 zugewandt. Die Oberseite 221a und die Unterseite 221b des zweiten Brückenkörpers 221 sind durch Seitenflächen miteinander verbunden, wobei zwei sich gegenüberliegende Seitenflächen die Stirnseiten 221c, 221d des Brückenkörpers 221 bilden und die verbliebenen sich gegenüberliegenden Seitenflächen die Schmalseiten 221e, 221f des Brückenkörpers 221 bilden. An der Unterseite 221b des Brückenkörpers 221 ist die Ausnehmung 222 zur Aufnahme des zweiten Abschnittes 122 des zweiten Fixierungselementes 12 ausgebildet. Die Ausnehmung 222 erstreckt sich dabei bis zur Schmalseite 221f des Brückenkörpers 221. An dieser Schmalseite 221f ist die Ausnehmung 222 offen, um ein Einbringen des zweiten Abschnittes 122 des zweiten Fixierungselementes 12 unter Herstellung einer lösbaren Verbindung zu ermöglichen.

Das zweite Brückenelement 22 weist eine Befestigungsbohrung 223 auf, die sich von seiner Oberseite 211a bis zu seiner Unterseite 211b erstreckt. Sie endet dabei an der Ausnehmung 222. Durch die Befestigungsbohrung 223 und eine korrespondierende Bohrung 125, die im zweiten Abschnitt 122 des zweiten Fixierungselementes 12 ausgebildet ist, ist eine Schraube als zweites Befestigungsmittel geführt, mit deren Hilfe das zweite Brückenelement 22 mit dem zweiten Fixierungselement 12 lösbar verbunden ist. Die Verwendung einer Schraube erleichtert das Lösen der Verbindung. Dazu weisen die Befestigungsbohrung 223 und die korrespondierende Bohrung 125 jeweils ein Innengewinde (nicht gezeigt) auf. Die Verbindung kann jedoch auch auf andere Weise hergestellt werden.

Das zweite Brückenelement 22 weist eine Gewindebohrung 224 auf, die sich von der ersten Stirnseite 221c zu der zweiten Stirnseite 221d des Brückenkörpers 221 erstreckt. Die Gewindebohrung 224 weist ein Innengewinde (nicht gezeigt) auf. Das Innengewinde, das in der Gewindebohrung 224 des zweiten Brückenelements 22 ausgebildet ist, ist gegenläufig zum Innengewinde, das in der Gewindebohrung 214 des ersten Brückenelements 21 ausgebildet ist. Durch die Gewindebohrung 224 ist der Distraktionszylinder 31 geführt, der ein Außengewinde (nicht gezeigt) aufweist, das in das Innengewinde der Gewindebohrung 224 eingreift. Das zweite Brückenelemente 22 weist ferner zwei Führungsbohrungen 225, 226 auf, die sich ebenfalls von der ersten Stirnseite 221c zu der zweiten Stirnseite 221d des Brückenkörpers 221 erstrecken. Die Längsachsen der beiden Führungsbohrungen 225, 226 liegen parallel zur Längsachse der Gewindebohrung 224. Die beiden Führungsbohrungen 225, 226 sind von der Gewindebohrung 224 beabstandet. Die Längsachse der Gewindebohrung 224 liegt auf der Längsachse des Distraktionszylinders 31, wenn der Distraktionszylinder 31 durch die Gewindebohrung 224 geführt ist. Durch die Führungsbohrung 225 ist der Führungsstift 41, durch die Führungsbohrung 226 ist der Führungsstift 42 geführt. Weder die Führungsbohrungen 225, 226 noch die Führungsstifte 41, 42 weisen Gewinde auf.

Der Distraktionszylinder 31 weist zwei gegenläufige Außengewinde auf, von denen eines in das Innengewinde, das in der Gewindebohrung 214 des ersten Brückenelements 21 ausgebildet ist, und das andere in das Innengewinde, das in der Gewindebohrung 224 des zweiten Brückenelements 22 ausgebildet ist, eingreift. Durch Drehen des Stellelementes 32 wird der Distraktionszylinder 31 um seine Längsachse L gedreht (Pfeil A in Fig. 1), wodurch der Abstand zwischen den beiden Brückenelementen 21, 22, bezogen auf die Längsachse L, verändert wird (Pfeil B). Bei einer Distraktion wird der Abstand in der Regel vergrößert, zur Korrektur einer zu starken Vergrößerung des Abstandes kann auch eine Verringerung des Abstandes vorgesehen sein. Mittels eines Stoppelementes 33, das in eine Ausnehmung des Stellelementes 32 eingesetzt werden kann, ist es möglich, eine unerwünschte Drehung des Distraktionszylinders 31 zu verhindern. Wenn eine Drehung des Distraktionszylinders 31 vom Behandler vorgenommen werden soll, wird das Stoppelement 33 aus der Ausnehmung entnommen und nach Vollenden der gewünschten Drehung in diese oder eine andere Ausnehmung des Stellelementes 32 eingesetzt. Es ist in Fig. 1 zu erkennen, dass das Stellelement 32 mehrere Ausnehmungen aufweist, die in Umfangsrichtung äquidistant zueinander an der äußeren Mantelfläche ausgebildet sind.

Der in Fig. 5 gezeigte Konsolidierungssteg 51 ersetzt den Schraubapparat nach Ende der Distraktionsphase. Am Ende der Distraktionsphase ist eine Veränderung des Abstandes zwischen den Brückenelementen 21, 22 nicht mehr erforderlich, stattdessen soll der mittels der Distraktionseinrichtung eingestellte Abstand der beiden Knochensegmente, an denen die Fixierungselemente 11, 12 befestigt sind, durch die Konsolidierungseinrichtung gehalten werden. Dazu wird der Konsolidierungssteg 51 an den beiden Fixierungselementen 11, 12 befestigt.

Der Konsolidierungssteg 51 weist eine quaderförmige Grundform mit einer oberen Flächenseite 51a und einer unteren Flächenseite 51b auf, die voneinander beabstandet sind und parallel zueinander verlaufen. Die untere Flächenseite 51b ist in der Konsolidierungsphase den beiden Fixierungselementen 11, 12, die am Kiefer des Patienten befestigt sind, zugewandt. Die obere Flächenseite 51a und die untere Flächenseite 51b des Konsolidierungssteges 51 sind durch Seitenflächen miteinander verbunden, wobei zwei sich gegenüberliegende Seitenflächen die Stirnseiten 51c, 51d des Konsolidierungssteges 51 bilden und die verbliebenen sich gegenüberliegenden Seitenflächen die Schmalseiten 51e, 51f des Konsolidierungssteges 51 bilden. An der unteren Flächenseite 51b sind eine erste Ausnehmung 511 zur Aufnahme des zweiten Abschnittes 112 des ersten Fixierungselementes 11 und eine zweite Ausnehmung 512 zur Aufnahme des zweiten Abschnittes 122 des zweiten Fixierungselementes 12 ausgebildet.

Während der Konsolidierungsphase greift in die erste Ausnehmung 511 des Konsolidierungssteges 51 der zweite Abschnitt 112 des ersten Fixierungselementes 11 ein, während in die zweite Ausnehmung 512 des Konsolidierungssteges 51 der zweite Abschnitt 122 des zweiten Fixierungselementes 12 eingreift. Die Gestalt der ersten Ausnehmung 511 entspricht der Gestalt der Ausnehmung 212 des ersten Brückenelementes 21, und die Gestalt der zweiten Ausnehmung 512 entspricht der Gestalt der Ausnehmung 222 des zweiten Brückenelementes 22. Auf diese Weise kann ein leichter Austausch des Schraubapparates, der die beiden Brückenelemente 21, 22 aufweist, gegen den Konsolidierungssteg 51 erreicht werden.

Der Konsolidierungssteg 51 weist eine erste Befestigungsbohrung 521 und eine zweite Befestigungsbohrung 522 auf, die sich von seiner oberen Flächenseite 51a bis zu seiner unteren Flächenseite 51b erstrecken. Dabei endet die erste Befestigungsbohrung 521 an der ersten Ausnehmung 511, die zweite Befestigungsbohrung 522 an der zweiten Ausnehmung 512. Durch die erste Befestigungsbohrung 521 und die korrespondierende Bohrung 115, die im zweiten Abschnitt 112 des ersten Fixierungselementes 11 ausgebildet ist, ist in der Konsolidierungsphase eine Schraube, die als zweites Befestigungsmittel dient, geführt, mit deren Hilfe der Konsolidierungssteg 51 mit dem ersten Fixierungselement 11 lösbar verbunden ist. Dazu weisen die erste Befestigungsbohrung 521 und die korrespondierende Bohrung 115 jeweils ein Innengewinde (nicht gezeigt) auf. Außerdem ist durch die zweite Befestigungsbohrung 522 und die korrespondierende Bohrung, die im zweiten Abschnitt 122 des zweiten Fixierungselementes 12 ausgebildet ist, in der Konsolidierungsphase eine Schraube, die als weiteres zweites Befestigungsmittel dient, geführt, mit deren Hilfe der Konsolidierungssteg 51 mit dem zweiten Fixierungselement 12 lösbar verbunden ist. Dazu weisen die zweite Befestigungsbohrung 522 und die korrespondierende Bohrung 125 jeweils ein Innengewinde (nicht gezeigt) auf. Die beiden zweiten Befestigungsmittel können die Befestigungsmittel sein, die während der Distraktionsphase zum Befestigen des ersten Brückenelementes 21 am ersten Fixierungselement 11 und zum Befestigen des zweiten Brückenelementes 22 am ersten Fixierungselement 12 verwendet wurden. Die Verbindungen zwischen den beiden Fixierungselementen 11, 12 und dem Konsolidierungssteg 51 können jedoch auch auf andere Weise hergestellt werden.

Fig. 7 zeigt die Konsolidierungseinrichtung, die mittels des erfindungsgemäßen Distraktionssystems 1 hergestellt wird. Dabei ist der Konsolidierungssteg 51 an den beiden Fixierungselementen 11, 12 lösbar befestigt, die wiederum am Unterkiefer 5 des Patienten mittels der Schrauben 2 befestigt sind. Das erste Fixierungselement 11 ist an einem ersten Kiefersegment 3 befestigt, und das zweite Fixierungselement 12 ist an einem anderen Kiefersegment 4 befestigt.

### Literatur

1. Becker MJ. Mandibular symphysis (medial suture) closure in modern Homo sapiens: preliminary evidence from archaeological populations. American journal of physical anthropology. 1986;69(4):499-501.
2. Beecher RM. Function and fusion at the mandibular symphysis. American journal of physical anthropology. 1977;47(2):325-35.
3. Lieberman DE, Crompton AW. Why fuse the mandibular symphysis? A comparative analysis. American journal of physical anthropology. 2000;112(4):517-40.
4. Brenner E. What is Meant by "Symphysis of the Mandible". lnf Orthod Kieferor. 2013;45(4):260-1.
5. Sperber GH, Sperber SM. Craniofacial embryogenetics and development. Raleigh, North Carolina: PMPH USA; 2018.
6. Moorrees CF, Gron AM, Lebret LM, Yen PK, Frohlich FJ. Growth studies of the dentition: a review. American journal of orthodontics. 1969;55(6):600-16.
7. Stöckli PW. 2. Postnataler Wachstumsverlauf, Kieferwachstum und Entwicklung der Dentition. In: Hotz RP, editor. Zahnmedizin bei Kindern und Jugendlichen. Stuttgart: Thieme; 1976.
8. Rakosi T, Jonas 1. Kieferorthopädische Diagnostik. Stuttgart-New York: Thieme; 1989.
9. Miethke RR. Schädelentwicklung. In: Diedrich P, editor. Kieferorthopädie 1. 4. Auflage. München, Jena: Urban&Fischer; 2000. p. 19-41.
10. Dausch-Neumann D. [Angle of the jaw in eugnathic and progenic dentition]. Fortschritte der Kiefe ro rtho pad ie. 1985 ;46( 5) :358-68.
11. Codivilla A. The classic: On the means of lengthening, in the lower limbs, the muscles and tissues which are shortened through deformity. 1905. Clinical orthopaedics and related research. 2008;466(12):2903-9.
12. Guerrero CA, Bell WH, Contasti GI, Rodriguez AM. Mandibular widening by intraoral distraction osteogenesis. The British journal of oral & maxillofacial surgery. 1997;35(6):383-92.
13. Evrard A, Tepedino M, Cattaneo PM, Cornelis MA. Which factors influence orthodontists in their decision to extract? A questionnaire survey. Journal of clinical and experimental dentistry. 2019;11(5):e432-e8.
14. Seeberger R, Kater W, Davids R, Thiele OC, Edelmann B, Hofele C, et al. Changes in the mandibular and dento-alveolar structures by the use of tooth borne mandibular symphyseal distraction devices. Journal of cranio-maxillo-facial surgery : official publication of the European Association for Cranio-Maxillo-Facial Surgery. 2011;39(3):177-81.
15. Jung SV, Shin SV, Lee KH, Eun YG, Lee YC, Kirn SW. Analysis of mandibular structure using 30 facial computed tomography. Otolaryngology--head and neck surgery : official journal of American Academy of Otolaryngology-Head and Neck Surgery. 2014;151(5):760-4.
16. Alkan A, Ozer M, Bas B, Bayram M, Celebi N, lnal S, et al. Mandibular symphyseal distraction osteogenesis: review of three techniques. International journal of oral and maxillofacial surgery. 2007;36(2):111-7.
17. Raoul G, Wojcik T, Ferri J. Outcome of mandibular symphyseal distraction osteogenesis with bone-borne devices. The Journal of craniofacial surgery. 2009;20(2):488-93.
18. Uckan S, Veziroglu F, Arman A. Unexpected breakage of mandibular midline distraction device: case report. Oral surgery, oral medicine, oral pathology, oral radiology, and endodontics. 2006;102(6):e21-5.

### Bezugszeichenliste

- 1: Distraktionssystem
- 2: Schraube
- 3: Kiefersegment
- 4: Kiefersegment
- 5: Unterkiefer

- 11: erstes Fixierungselement
- 111: erster Abschnitt
- 112: zweiter Abschnitt
- 113: Verbindungsabschnitt
- 114: Öffnung
- 115: korrespondierende Bohrung
- 12: zweites Fixierungselement
- 121: erster Abschnitt
- 122: zweiter Abschnitt
- 123: Verbindungsabschnitt
- 124: Öffnung
- 125: korrespondierende Bohrung
- 21: erstes Brückenelement
- 211: Brückenkörper
- 211a: Oberseite
- 211b: Unterseite
- 211c: Stirnseite
- 211d: Stirnseite
- 211e: Schmalseite
- 211f: Schmalseite
- 212: Ausnehmung
- 213: Befestigungsbohrung
- 214: Gewindebohrung
- 215: Führungsbohrung
- 216: Führungsbohrung
- 22: zweites Brückenelement
- 221: Brückenkörper
- 221a: Oberseite
- 221b: Unterseite
- 221c: Stirnseite
- 221d: Stirnseite
- 221e: Schmalseite
- 221f: Schmalseite
- 222: Ausnehmung
- 223: Befestigungsbohrung
- 224: Gewindebohrung
- 225: Führungsbohrung
- 226: Führungsbohrung
- 31: Distraktionszylinder
- 32: Stellelement
- 33: Stoppelement
- 41: Führungsstift
- 42: Führungsstift
- 51: Konsolidierungssteg
- 51a: obere Flächenseite
- 51b: untere Flächenseite
- 51c: Stirnseite
- 51d: Stirnseite
- 51e: Schmalseite
- 51f: Schmalseite
- 511: erste Ausnehmung
- 512: zweite Ausnehmung
- 521: Befestigungsbohrung
- 522: Befestigungsbohrung

## Patentansprüche

1. Distraktionssystem, insbesondere zur transversalen Erweiterung eines Kiefers, aufweisend ein erstes Fixierungselement (11) zur Befestigung am Kiefer sowie ein zweites Fixierungselement (12) zur Befestigung am Kiefer, wobei das Distraktionssystem (1) ferner ein erstes Brückenelement (21) zur lösbaren Befestigung an dem ersten Fixierungselement (11), ein zweites Brückenelement (22) zur lösbaren Befestigung an dem zweiten Fixierungselement (12) und einen Distraktionszylinder (31) zum Verändern des Abstandes zwischen dem ersten Brückenelement (21) und dem zweiten Brückenelement (22) aufweist, **dadurch gekennzeichnet, dass** das Distraktionssystem (1) ferner einen Konsolidierungssteg (51) zur lösbaren Befestigung an dem ersten Fixierungselement (11) und dem zweiten Fixierungselement (12) unter Ersatz des ersten Brückenelementes (21), des zweiten Brückenelementes (22) und des Distraktionszylinders (31) aufweist.

2. Distraktionssystem nach Anspruch 1, **dadurch gekennzeichnet, dass** das erste und das zweite Brückenelement (21, 22) jeweils einen Brückenkörper (211, 221) mit einer Gewindebohrung (214) zur Einführung des Distraktionszylinders (31) aufweisen.

3. Distraktionssystem nach Anspruch 2, **dadurch gekennzeichnet, dass** der Distraktionszylinder (31) zwei gegenläufige Außengewinde zum Eingriff in Innengewinde aufweist, die in der Gewindebohrung (214) des ersten Brückenelementes (21) und in der Gewindebohrung des zweiten Brückenelementes (22) ausgebildet sind.

4. Distraktionssystem nach einem der vorstehenden Ansprüche, **dadurch gekennzeichnet, dass** er zumindest einen Führungsstift (41, 42) zum Führen des ersten Brückenelementes (21) und des zweiten Brückenelementes (22) aufweist und dass das erste Brückenelement (21) und das zweite Brückenelement (22) jeweils eine Führungsbohrung (215, 216) zur Aufnahme des Führungsstiftes (41, 42) aufweisen.

5. Distraktionssystem nach Anspruch 4, **dadurch gekennzeichnet, dass** es zwei Führungsstifte (41, 42) aufweist, deren Längsachsen parallel zueinander und zur Längsachse (L) des Distraktionszylinders (31) verlaufen, wobei der Distraktionszylinder (31) zwischen den beiden Führungsstiften (41, 42) angeordnet ist.

6. Distraktionssystem nach einem der vorstehenden Ansprüche, **dadurch gekennzeichnet, dass** das erste Fixierungselement (11) und das zweite Fixierungselement (12) jeweils einen ersten Abschnitt (111, 121) aufweisen, in dem zumindest eine Öffnung (114, 124) zur Durchführung eines Befestigungsmittels ausgebildet ist.

7. Distraktionssystem nach einem der vorstehenden Ansprüche, **dadurch gekennzeichnet, dass** das erste Fixierungselement (11) und das zweite Fixierungselement (12) jeweils einen zweiten Abschnitt (112, 122) zur lösbaren Befestigung entweder an einem der beiden Brückenelemente (21, 22) oder an dem Konsolidierungssteg (51) aufweisen.

8. Distraktionssystem nach Anspruch 7, **dadurch gekennzeichnet, dass** das erste Brückenelement (21) und das zweite Brückenelement (22) jeweils eine Ausnehmung (212, 222) zur Aufnahme des zweiten Abschnittes (112, 122) eines der beiden Fixierungselemente (11, 12) aufweisen.

9. Distraktionssystem nach Anspruch 7 oder Anspruch 8, **dadurch gekennzeichnet, dass** der Konsolidierungssteg (51) eine erste Ausnehmung (511) zur Aufnahme des zweiten Abschnittes (112) des ersten Fixierungselementes (11) und eine zweite Ausnehmung (512) zur Aufnahme des zweiten Abschnittes (122) des zweiten Fixierungselementes (12) aufweist.

10. Distraktionssystem nach einem der Ansprüche 7 bis 9, **dadurch gekennzeichnet, dass** das erste Fixierungselement (11) und das zweite Fixierungselement (12) jeweils einen Verbindungsabschnitt (113, 123) aufweisen, der den ersten Abschnitt (111, 121) eines Fixierungselementes (11, 12) mit dem zweiten Abschnitt (112, 122) dieses Fixierungselementes (11, 12) verbindet.

11. Distraktionsset zur Herstellung einer Distraktionseinrichtung, insbesondere einer Distraktionseinrichtung zur transversalen Erweiterung eines Kiefers, und zur Herstellung einer Konsolidierungseinrichtung, wobei das Distraktionsset aufweist:
- ein erstes Fixierungselement (11) zur Befestigung am Kiefer sowie ein zweites Fixierungselement (12) zur Befestigung am Kiefer;
- zur Herstellung der Distraktionseinrichtung ein erstes Brückenelement (21) zur lösbaren Befestigung an dem ersten Fixierungselement (11), ein zweites Brückenelement (22) zur lösbaren Befestigung an dem zweiten Fixierungselement (12) und einen Distraktionszylinder (31) zum Verändern des Abstandes zwischen dem ersten Brückenelement (21) und dem zweiten Brückenelement (22); und
- zur Herstellung der Konsolidierungseinrichtung einen Konsolidierungssteg (51) zur lösbaren Befestigung an dem ersten Fixierungselement (11) und an dem zweiten Fixierungselement (12).

12. Distraktionsset nach Anspruch 11, **dadurch gekennzeichnet, dass** es Befestigungsmittel zur Befestigung des ersten Fixierungselementes (11) und des zweiten Fixierungselementes (12) am Kiefer aufweist.

13. Distraktionsset nach Anspruch 11 oder Anspruch 12, **dadurch gekennzeichnet, dass** es zweite Befestigungsmittel zur lösbaren Befestigung des ersten Fixierungselements (11) an dem ersten Brückenelement (21) oder an dem Konsolidierungssteg (51) und zur lösbaren Befestigung des zweiten Fixierungselements (12) an dem zweiten Brückenelement (22) oder an dem Konsolidierungssteg (51) aufweist.

14. Distraktionsset nach einem der Ansprüche 11 bis 13, **dadurch gekennzeichnet, dass** es zumindest einen Führungsstift (41, 42) zum Führen des ersten Brückenelementes (21) und des zweiten Brückenelementes (22) aufweist.
